# EUROPEAN PATENT APPLICATION

(11) **EP 4 494 674 A1**
(43) Date of publication of application: **22.01.2025**
(21) Application number: 22931819.1
(22) Date of filing: 30.11.2022
(51) Int. Cl.: A61M 5/158, A61M 5/14, A61M 39/10

(54) **PRESSURE CONVERSION DEVICE AND INFUSION CONNECTION JOINT**

(30) Priority: 18.03.2022 CN 202210268110
(71) Applicant: Deng, Sanming, Xi'an, Shaanxi 710016 (CN)
(72) Inventor: Deng, Sanming, Xi'an, Shaanxi 710016 (CN)
(74) Representative: JD&P Patent Attorneys Joanna Dargiewicz & Partners
(86) International application number: PCT/CN2022/135231
(87) International publication number: WO 2023/173808

(57) **Abstract**

A pressure changing device and an infusion connector are provided. The pressure changing device includes a pressure changing member. When the pressure changing device of the present invention is connected to an infusion head or a syringe head, a negative pressure is generated in the pressure changing device, which draws thrombus from a connected indwelling needle into an indwelling needle holder or the pressure changing device, thereby preventing the thrombus from re-entering the blood vessel of a patient through the indwelling needle. Then the negative pressure disappears, and liquid can flow into the blood vessel normally through the indwelling needle. When removing the infusion head or the syringe head, the pressure changing device generates a positive pressure for a certain period of time, pushing the liquid in the indwelling needle into the blood vessel, thereby avoiding blood reflux and reducing the formation of thrombus in the indwelling needle.

## Description

### Field of the Invention

The present invention relates to a technical field of medical devices, and more particularly to a pressure changing device and an infusion connector.

### Background of the Invention

Indwelling needles are commonly used in medical clinics to prevent repeated punctures, simplify the operation, and minimize patient suffering. The indwelling needle is routinely connected to needleless connector and infusion pathway. Conventionally, the recommended needleless infusion connector is a positive pressure connector, which produces a certain positive pressure at the moment of removing the infusion pathway, thereby pushing the liquid inside the indwelling needle into the blood vessel, avoiding blood reflux, and preventing the formation of thrombus inside the indwelling needle. However, it is impossible to completely prevent blood from flowing back into the soft outer casing of the indwelling needle when infusion is stopped, resulting in thrombus that blocks the needle. Moreover, the thrombus may enter the blood vessels along with fluid when it is re-infused.

A more reasonable approach in the prior art is to use a syringe to aspirate the blood and thrombus together through the infusion connector connected to the indwelling needle. Then saline is used to rinse the indwelling needle before re-infusing the fluid through the indwelling needle. Although the thrombus in the indwelling needle will be sucked out, or retained in the indwelling needle or the infusion connector, which reduces the chance of the thrombus entering the human blood vessel through the indwelling needle, the blood that is sucked out increases the risk of hepatitis B, AIDS and other infections for the medical personnel, as well as increases the hospital's investment in the treatment of medical wastes.

### Summary of the Invention

In order to solve the above technical problems, an object of the present invention is to provide a pressure changing device and an infusion connector, which changes a pressure with a pressure changing member. When the pressure changing device of the present invention is connected to an infusion head or a syringe head, a negative pressure is generated in the pressure changing device, which draws thrombus from a connected indwelling needle into an indwelling needle holder or the pressure changing device, thereby preventing the thrombus from re-entering the blood vessel of a patient through the indwelling needle. Then the negative pressure disappears, and liquid can flow into the blood vessel normally through the indwelling needle. When removing the infusion head or the syringe head, the pressure changing device generates a positive pressure for a certain period of time, pushing the liquid in the indwelling needle into the blood vessel, thereby avoiding blood reflux and reducing the formation of thrombus in the indwelling needle.

Accordingly, in order to accomplish the above objects, the present invention provides:
a pressure changing device for an infusion connector, comprising: a pressure changing member, wherein the pressure changing member is switchable between a natural state and a compressed state; in the natural state, the pressure changing member seals a chamber of the pressure changing device; with an external pressure, the pressure changing member is switched from the natural state to the compressed state, and the chamber of the pressure changing device is then communicates with an external fluid and a negative pressure is generated within the pressure changing device for a certain period of time; after the external pressure is removed, the pressure changing member is switched from the compressed state to the naturel state, and a positive pressure is generated within the pressure changing device for a certain period of time.

When inserting the infusion head or the syringe head into the pressure changing device, the pressure changing member is compressed by the infusion head or syringe head, so that the pressure changing device generates a negative pressure for a certain period of time, and the thrombus inside the indwelling needle is sucked into the pressure changing device. Then the negative pressure disappears, and liquid can smoothly pass through the pressure changing device to enter the indwelling needle. When removing the infusion head or the syringe head, the pressure changing member stretches out under a resilience force and switches from the compressed state to the natural state. The pressure changing device generates a positive pressure for a certain period of time, which pushes the liquid inside the indwelling needle into blood vessel, avoids blood reflux, and reduces the formation of thrombus inside the indwelling needle.

Preferably, the pressure changing member comprises a coupling assembly, a resilient structure, a mobile body and a connecting member.

The mobile body is disposed at an outflow end (outflow tract end) of the pressure changing device; one end of the connecting member is proximate to the outflow end, and is directly or indirectly connected to the mobile body; the other end of the connecting member is proximate to an inflow end (inflow tract end) of the pressure changing device, and is directly or indirectly connected to the coupling assembly.

When inserting the infusion head or the syringe head into the pressure changing device, the coupling assembly moves with the infusion head from the inflow end to the outflow end of the pressure changing device. Under the action of the connecting member, the mobile body moves in the opposite direction (from the outflow end to the inflow end of the pressure changing device). The movement of the mobile body and/or the connecting member drives the resilient structure to move, thereby compressing or stretching the resilient structure. The movement of the mobile body from the outflow end to the inflow end reduces the local liquid pressure within the housing of the pressure changing device, which is less than the pressure within the indwelling needle, thus generating a negative pressure that pulls the liquid from the indwelling needle towards the pressure changing device. Then the negative pressure disappears, and the liquid can smoothly pass through the pressure changing device and enter the indwelling needle. When removing the infusion head or the syringe head, the infusion head or the syringe head drives the connecting member to move from the outflow end to the inflow end, and the resilient structure restores to an initial state, which drives the connecting member back to an initial state and position, and moves the mobile body and the connector assembly in the opposite direction (from the inflow end to the outflow end). The movement of the mobile body from the inflow end to the outflow end increases the local liquid pressure within the housing of the pressure changing device, making it greater than the pressure within the indwelling needle, thus generating a positive pressure that pushes the liquid from the pressure changing device means to the indwelling needle, avoiding blood from entering the indwelling needle at the moment of removing the infusion head or the syringe head, and avoiding the formation of thrombus.

Preferably, the mobile body comprises one or more components to move as a whole or in part, or the mobile body changes a local pressure by deformation.

Preferably, one end of the resilient structure is attached to the coupling assembly or to a fixed position in the pressure changing device, and the other end is connected directly or indirectly to the mobile body or/and to the connecting member; or a middle portion of the resilient structure is fixed to the coupling assembly or to the fixed position in the pressure changing device, and two ends of the resilient structure are directly or indirectly connected to the mobile body and the connecting member, respectively.

Preferably, the resilient structure is chopped into one group or multiple groups; a resilient force of the resilient structure is a retraction force of a spring or a resilience force of a compressed elastic material, or a resilience force generated by a deformed chamber wall of an inelastic material; the resilient structure forms at least part of a liquid lumen, or forms a liquid lumen with an additional closed body; liquid enters an indwelling needle through the liquid lumen.

The resilient structure provides a force to drive the mobile body back to its initial state, and also forms a liquid flow path to prevent the liquid from coming into contact with other devices, thereby reducing the contact surface of the liquid and avoiding infection.

Preferably, the coupling assembly may move together with the infusion head or the syringe head in the same direction. The coupling assembly may be a rubber valve.

Preferably, the connecting member comprises a pivot point and a strip of soft or/and rigid bars around or through the pivot point.

Preferably, the pivot point is a fixed point or one end of a fixed surface provided within the pressure changing device, which also plays a fixing role within the pressure changing device.

Preferably, the inflow end of the connecting member may be connected directly or indirectly to the coupling assembly of the pressure changing device.

Preferably, a resilient force of the resilient structure is a retraction force of a spring or a resilience force of a compressed elastic material, or a resilience force generated by a deformed chamber wall of an inelastic material; the resilient structure forms at least part of a liquid lumen. Preferably, one end of the connecting member is directly or indirectly connected to the coupling assembly, and the other end is directly or indirectly connected to the mobile body. Movement of the coupling assembly drives the connecting member to move, which in turn drives the mobile body to move. The movement of the mobile body generates the negative or positive pressure within the liquid chamber of the pressure changing device.

Preferably, a fixing member, such as a beam, is provided within the pressure changing device.

Preferably, the resilient structure is provided between the mobile body and a housing of the pressure changing device or a fixed point within the housing, facilitating the mobile body to return its initial state.

Preferably, the resilient structure is capable of separating a liquid flow path.

Preferably, an exhaust pathway may be provided within the beam.

Preferably, at least two of the coupling assembly, the connecting member, the mobile body and the resilient structure are integrally molded.

Preferably, a closed body may be added within the pressure changing device to form a complete fluid pathway, preventing the fluid from coming into contact with other devices, reducing the contact surface of the fluid, and avoiding infection.

Preferably, the housing of the pressure changing device comprises a chamber, an inflow tract opening and an outflow tract opening. The outflow tract opening can be connected to a peripheral venous indwelling needle, a central venous indwelling needle, and a three-way valve port. The inflow tract opening can be connected to and locked with a syringe, an infusion set, and a blood transfusion set.

Preferably, a flap is provided at the inflow tract opening of the housing. When inserting the infusion head, the flap is opened by the infusion head. When removing the infusion head, the flap is closed by the resilient structure, thereby closing the inflow tract opening. Preferably, the pressure changing member comprises a rubber valve, a resilient structure and a connecting member, wherein the resilient structure is spiral or semi-circular; and deformation of the resilient structure switches negative and positive pressures in a local liquid chamber.

When the infusion head is inserted into the pressure changing device, it drives the coupling assembly and one end of the connecting member to move toward the outflow end, and drives the mobile body and the resilient structure to move gradually towards the inflow end, thereby changing the local liquid pressure, wherein the local liquid pressure is less than the liquid pressure in the indwelling needle, so that the liquid moves from the indwelling needle towards the pressure changing device. At the same time, a curved shape of the resilient structure is opened to connect the liquid pathway. After the infusion head is inserted into the pressure changing device and locked, the liquid pathway remains opened, and drug and liquid can enter the indwelling needle and body blood vessel through the device of the present invention.

When the infusion head is removed from the pressure changing device, the resilience force of the mobile body and the resilient structure restores the curved shape of the resilient structure, so as to change the local liquid pressure, generate a positive pressure, and drive the device of the present invention back to its initial state.

The present invention further provides an infusion connector, comprising the above pressure changing device.

Preferably, the pressure changing device and the infusion connector share the same housing.

The present invention requires no more than the conventional operation. During infusion, the pressure changing device can first apply a certain negative pressure on the indwelling needle to draw the thrombus inside a soft casing of the indwelling needle into a needle holder of the indwelling needle and the pressure changing device, preventing the thrombus from entering the blood vessel of a patient through the indwelling needle. Meanwhile, after the infusion, the pressure changing device can generate a positive pressure to push the liquid inside indwelling needle into the blood vessel, avoiding blood reflux and reducing the formation of thrombus in the indwelling needle.

### Brief Description of the Drawings

FIGS. 1-2 are sketch views according to an embodiment 1 of the present invention;
FIGS. 3-4 are sketch views according to an embodiment 2 of the present invention;
FIGS. 5-6 are sketch views according to an embodiment 3 of the present invention; and
FIGS. 7-8 are sketch views according to an embodiment 4 of the present invention.

### Element reference:

1: pressure changing member; 1-1: connecting member; 1-1-1: inflow tract end of the connecting member; 1-1-2: outflow tract end of the connecting member; 1-2: mobile body; 1-3: resilient structure; 1-4: relative fixed point; 2: housing; 3: inflow tract opening; 4: outflow tract opening; 5: rubber valve; 6: beam; 7: first chamber; 8: positive end outflow tract; 9: infusion head; 10: flap; 11: sealing membrane; 12: connector angle.

### Detailed Description of the Invention

The technical solutions in the embodiments of the present invention will be clearly and completely described below in conjunction with the accompanying drawings. It will be apparent that the embodiments to be described are only a part of all the embodiments of the present invention. Components of the embodiments of the present invention, which are generally described and illustrated in the accompanying drawings herein, may be arranged and designed in a variety of configurations. Accordingly, the description of embodiments of the present invention that are illustrated by the accompanying drawings is not intended to limit the protection scope of the present invention, but are only preferred embodiments of the present invention. Based on the embodiments of the present invention, all other implementations obtained by those skilled in the art without creative work are within the protection scope of the present invention.

It should be noted that similar symbols and letters denote similar items in the accompanying drawings, so that once an item is defined in one drawing, it does not need to be further defined and explained in subsequent drawings. Also, in the description of the present invention, the terms "first", "second", etc. are used only for distinguishing and are not to be understood as indicating or implying relative importance.

### Embodiment 1

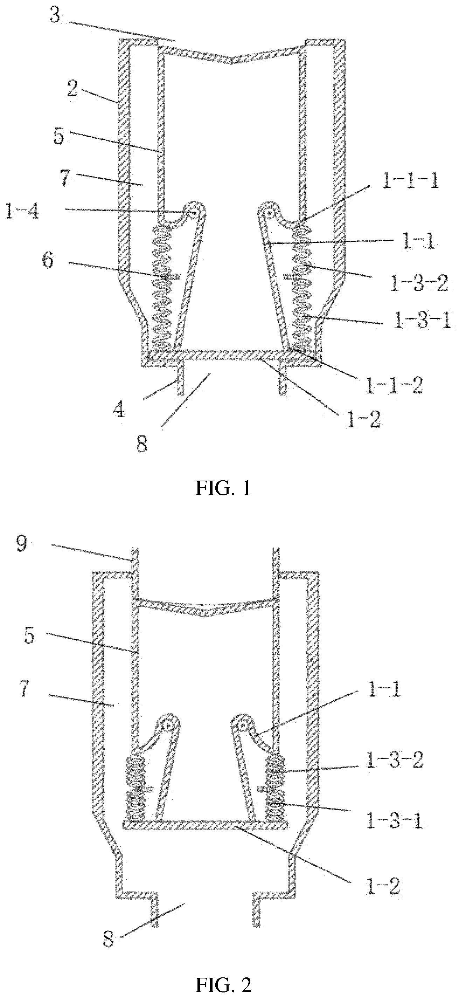

Referring to FIG. 1, the pressure changing device of the present invention comprises: a housing 2, an inflow tract opening 3, and an outflow tract opening 4. The outflow tract opening 4 can be arbitrarily connected to a peripheral venous indwelling needle, a central venous indwelling needle, and ports like a three-way valve. The inflow tract opening 3 can be connected to and locked with syringes, an infusion set, a blood transfusion set, and the like.

The housing 2 of the device of the present invention is closed and kept sterile inside in the absence of any connector.

A pressure changing member 1 of the present invention comprises: a rubber valve 5, a resilient structure 1-3, a mobile body 1-2 and a connecting member 1-1.

A top of the rubber valve 5 is provided with a convex point or a concave point so that the liquid can flow smoothly into the pressure changing device. The rubber valve 5 is connected to the connecting member 1-1, and the connecting member 1-1 bypasses a relative fixed point 1-4 and is connected to the mobile body 1-2. The mobile body 1-2 is close to an outflow tract of the pressure changing device and matches morphologically with part of a casing of the outflow tract, thereby sealing it.

A fixing point (beam 6) is provided within the pressure changing device, which plays a fixing role within the pressure changing device.

Another resilient member 1-3-2 may be arranged between the rubber valve 5 and the beam 6, which may facilitate the rubber valve 5 to restore its initial state. A resilient member 1-3-1 is provided between the mobile body 1-2 and the beam 6, wherein one end of the resilient member 1-3-1 is connected to the beam 6, and the other end is connected to the mobile body 1-2.

The resilient members 1-3-1 and 1-3-2 can separate a liquid flow path, and form a first chamber 7 with the housing of the device.

The mobile body 1-2 and the outflow tract opening 4 of the pressure changing device together form a positive end outflow tract 8 of the pressure changing device of the present invention. Since the mobile body 1-2 matches morphologically with part of the casing of the outflow tract, the positive end outflow tract 8 may not be directly connected to the first cavity 7. After the mobile body 1-2 is moved upward, it no longer matches with the casing of the outflow tract, and the positive end outflow tract communicates with the first chamber.

Referring to FIG. 2, when the infusion head 9 is inserted into the pressure changing device of the present invention, it drives the rubber valve 5 to move towards the outflow tract of the pressure changing device, and the liquid flows into the first chamber 7 by using the convex or concave point at the top of the rubber valve 5. The movement of the rubber valve 5 drives the connecting member to move, thereby driving the mobile body 1-2 to move towards the inflow tract of the pressure changing device, as well as to compress the resilient members 1-3-1 and 1-3-2. The movement of the mobile body 1-2 reduces a local liquid pressure in the positive end outflow tract 8, wherein the pressure is less than a pressure inside the indwelling needle, thereby generating a negative pressure. At this time, the liquid reversely flows from the indwelling needle towards the pressure changing device. After the mobile body 1-2 stops moving, the first chamber 7 communicates the positive end outflow tract 8 to form a complete liquid pathway, so that the liquid can smoothly enter the indwelling needle.

Referring to FIG. 1, after infusion, the infusion head 9 and the pressure changing device is unlocked. When the infusion head 9 is pulled out of the pressure changing device, it drives the rubber valve 5 to move towards the inflow tract, and the rubber valve 5 drives the connecting member 1-1 to move, so as to drive the mobile body 1-2 to move from the inflow tract to the outflow tract. At the same time, the previously compressed resilient members 1-3-1 and 1-3-2 return to the initial state, generating a resilient force that assist the rubber valve 5 to restore the initial state, as well as assist the mobile body 1-2 to move towards the positive end outflow tract 8 of the present invention. The movement of the mobile body 1-2 increases the liquid pressure in the positive end outflow tract 8, makes it greater than the pressure within the indwelling needle, thereby generating a positive pressure. At this point, the liquid flows from the device of the present invention towards the indwelling needle. After the mobile body 1-2 stops moving, it prevents the first chamber 7 from communicating with the positive end outflow tract 8, so that the pressure changing device returns to its natural state and is closed.

### Embodiment 2

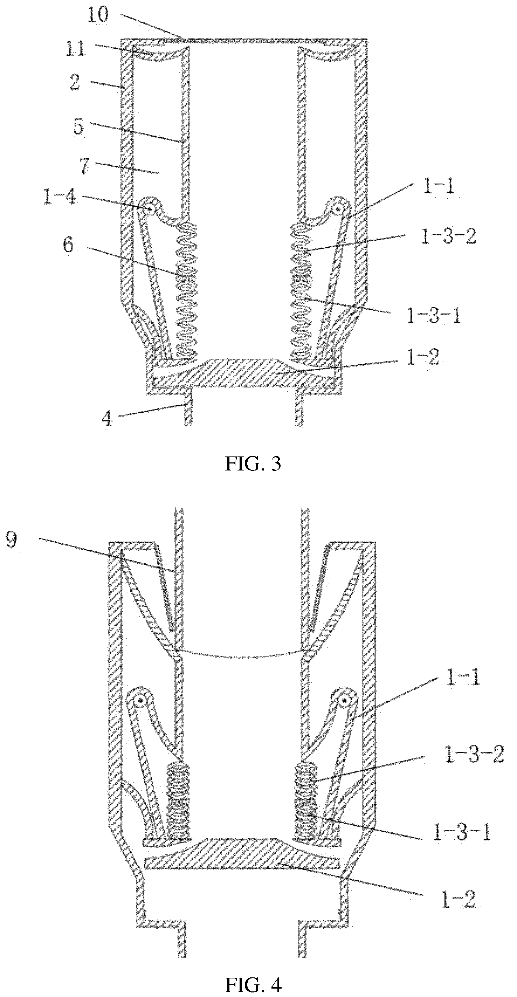

FIGS. 3-4 illustrate another embodiment of the present invention.

The embodiment 2 is different from the embodiment 1 in that:

A flap 10 is provided at the inflow tract opening of the housing. When inserting the infusion head 9, the flap 10 is opened by the infusion head. When removing the infusion head 9, the flap 10 is closed by the resilient structure, thereby closing the inflow tract opening. The coupling assembly 5 has a tubular structure in which the liquid can flow.

The mobile body 1-2 is provided at the outflow tract opening of the housing, which an aperture thereon. When the infusion head 9 is inserted into the pressure changing device, the mobile body 1-2 moves from the outflow tract to the inflow tract, thereby generating a negative pressure. The aperture on the mobile body 1-2 communicates the liquid pathway, and the outflow tract opening is open. When the infusion head 9 is removed, the mobile body 1-2 moves from the inflow tract to the outflow tract, thereby generating a positive pressure. The movement of mobile body 1-2 closes the outflow tract opening. Sealing membranes 11 are provided between the coupling assembly 5 and the housing as well as between the mobile body 1-2 and the housing, so as to prevent the liquid from coming into contact with other devices and avoid infection.

### Embodiment 3

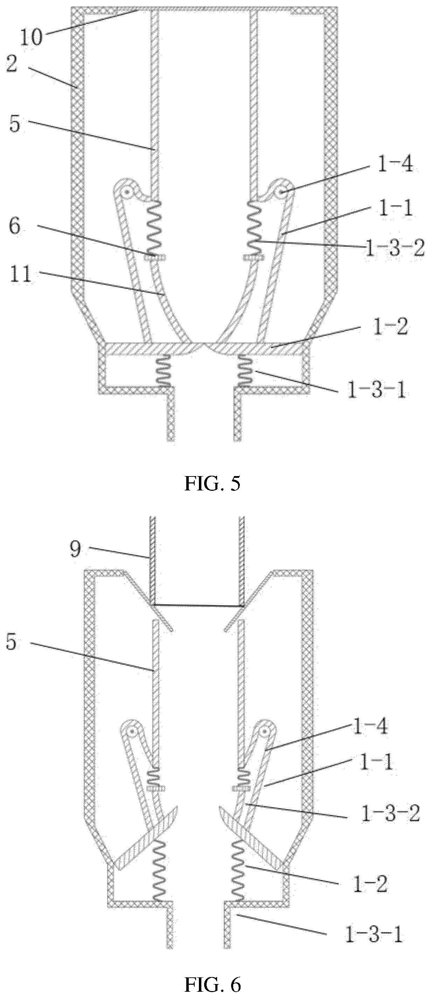

FIGS. 5-6 illustrate another embodiment of the present invention.

The embodiment 3 is different from the embodiment 2 in that:
The mobile body 1-2 is provided at the outflow tract opening of the housing, comprises one or more opening and closing members. One end of the mobile body is connected to the housing 2 and the other end is connected to the beam 6. The resilient member 1-3-1 is provided below the opening and closing members, and the resilient member 1-3-2 is provided between the beam and the rubber valve 5, so as to compose and close the liquid path. The sealing membrane 11 is provided between the mobile body 1-2 and the beam 6 to compose the liquid path.

### Embodiment 4

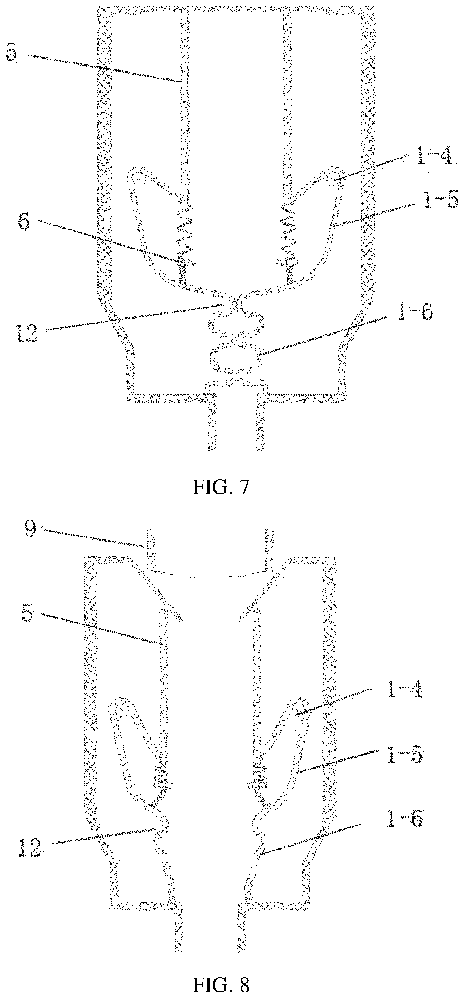

FIGS. 7-8 illustrate another embodiment of the present invention.

The embodiment 4 is different from the embodiment 3 in that (as shown in FIGS. 7-8):
The connecting member, the mobile body and the resilient structure are integrally designed, named a connecting transformation part 1-5, and a mobile body and resilient structure part 1-6, respectively.

The mobile body and resilient structure part 1-6 works as the mobile body, which changes the local liquid pressure. It also has the function of closing the liquid lumen, avoiding the liquid from contacting with other devices of the present invention, thus ensuring a sterile state, no drug impurity, and so on.

When the infusion head 9 is inserted into the pressure changing device, the coupling assembly 5 and one end of the connecting transformation part 1-5 move towards the outflow channel tract end, so as to gradually move the mobile body and resilient structure part 1-6 towards the inflow tract end to change the local liquid pressure. When the local liquid pressure is less than the liquid pressure in the indwelling needle, and the liquid moves from the indwelling needle to the pressure changing device. At the same time, a curved shape of the resilient structure is opened to connect the liquid pathway. After the infusion head 9 is inserted into the pressure changing device and locked, the liquid pathway remains opened, and drug and liquid can enter the indwelling needle and body blood vessel through the device of the present invention.

When the infusion head 9 is removed from the pressure changing device, the resilience force of the mobile body and resilient structure part 1-6 restores the curved shape of the resilient structure, so as to change the local liquid pressure, generate a positive pressure, and drive the device of the present invention back to its initial state.

Preferably, the present invention comprises a connector which works as the mobile body during stretching or compression of the pressure changing device. A connector angle is 12 degrees formed by itself, which may remain unchanged (as shown in FIGS. 7-8). Furthermore, all coupling/connection relationships mentioned herein do not refer solely to the direct connection between the members, but to the fact that a more optimal coupling structure can be formed by adding or reducing coupling auxiliaries, depending on the specific implementation. The various technical features of the present invention can be combined without conflict.

It is to be noted that according to the above disclosure and description, those skilled in the art to which the present invention belongs may also make changes and modifications to the above embodiments. Therefore, the present invention is not limited to the specific embodiments revealed and described above, and some equivalent modifications and changes to the present invention should also be protected by the present invention. In addition, although some specific terms are used herein, these terms are used only for the convenience of illustration and do not constitute any limitation of the present invention.

It can be easily understood by those skilled in the art that the foregoing description is only preferred embodiments of the present invention and is not intended to be limiting. Any modifications, equivalent substitutions, improvements, etc., made within the spirit and principles of the present invention shall be included in the claimed protection scope.

## Claims

1. A pressure changing device for an infusion connector, **characterized by** comprising: a pressure changing member, wherein the pressure changing member is switchable between a natural state and a compressed state; in the natural state, the pressure changing member seals a chamber of the pressure changing device; with an external pressure, the pressure changing member is switched from the natural state to the compressed state, and the chamber of the pressure changing device is then communicates with an external fluid and a negative pressure is generated within the pressure changing device for a certain period of time; after the external pressure is removed, the pressure changing member is switched from the compressed state to the naturel state, and a positive pressure is generated within the pressure changing device for a certain period of time.

2. The pressure changing device, as recited in claim 1, **characterized in that** the pressure changing member comprises a coupling assembly, a resilient structure, a mobile body and a connecting member, wherein the mobile body is disposed at an outflow end of the pressure changing device; one end of the connecting member is proximate to the outflow end, and is directly or indirectly connected to the mobile body; the other end of the connecting member is proximate to an inflow end of the pressure changing device, and is directly or indirectly connected to the coupling assembly.

3. The pressure changing device, as recited in claim 2, **characterized in that** the connecting member comprises a pivot point and a strip of soft or/and rigid bars around or through the pivot point.

4. The pressure changing device, as recited in claim 3, **characterized in that** the pivot point is a point close to the inflow end of the pressure changing device, or one side, or several points on the side.

5. The pressure changing device, as recited in claim 2, **characterized in that** one end of the resilient structure is attached to the coupling assembly or to a fixed position in the pressure changing device, and the other end is connected directly or indirectly to the mobile body or/and to the connecting member; or a middle portion of the resilient structure is fixed to the coupling assembly or to the fixed position in the pressure changing device, and two ends of the resilient structure are directly or indirectly connected to the mobile body and the connecting member, respectively.

6. The pressure changing device, as recited in claim 2, **characterized in that** the mobile body comprises one or more components to move as a whole or in part, or the mobile body changes a local pressure by deformation.

7. The pressure changing device, as recited in claim 2, **characterized in that** a fixing member is provided within a housing of the pressure changing device.

8. The pressure changing device, as recited in claim 5, **characterized in that** the resilient structure is chopped into one group or multiple groups; a resilient force of the resilient structure is a retraction force of a spring or a resilience force of a compressed elastic material, or a resilience force generated by a deformed chamber wall of an inelastic material; the resilient structure forms at least part of a liquid lumen, or forms a liquid lumen with an additional closed body; liquid enters an indwelling needle through the liquid lumen.

9. The pressure changing device, as recited in claim 2, **characterized in that** the coupling assembly is a rubber valve.

10. The pressure changing device, as recited in claim 2, **characterized in that** at least two of the coupling assembly, the connecting member, the mobile body and the resilient structure are integrally molded.

11. The pressure changing device, as recited in claim 1, **characterized in that** the pressure changing member comprises a rubber valve, a resilient structure and a connecting member, wherein the resilient structure is spiral or semi-circular; and deformation of the resilient structure switches negative and positive pressures in a local liquid chamber.

12. An infusion connector, **characterized by** comprising: the pressure changing device as recited in claims 1-11.
